(19) Europäisches Patentamt
European Patent Office
Office européen des brevets

(11) Veröffentlichungsnummer : **0 121 129**
**B1**

(12) **EUROPÄISCHE PATENTSCHRIFT**

(45) Veröffentlichungstag der Patentschrift : **01.02.89**

(51) Int. Cl.⁴ : **C 07 D207/44**, A 01 N 43/36

(21) Anmeldenummer : **84102339.3**

(22) Anmeldetag : **05.03.84**

# (54) 2,5-Dihydro-5-arylimino-pyrrolderivate, Verfahren zu ihrer Herstellung und ihre Verwendung zur Bekämpfung unerwünschten Pflanzenwuchses.

(30) Priorität : **09.03.83 DE 3308297**

(43) Veröffentlichungstag der Anmeldung : **10.10.84 Patentblatt 84/41**

(45) Bekanntmachung des Hinweises auf die Patenterteilung : **01.02.89 Patentblatt 89/05**

(84) Benannte Vertragsstaaten : **CH DE FR GB IT LI**

(56) Entgegenhaltungen :
**US-A- 3 072 673**
**US-A- 4 064 140**

(73) Patentinhaber : **BASF Aktiengesellschaft**
**Carl-Bosch-Strasse 38**
**D-6700 Ludwigshafen (DE)**

(72) Erfinder : **Becker, Rainer, Dr.**
**Im Haseneck 22**
**D-6702 Bad Duerkheim (DE)**
Erfinder : **Wuerzer, Bruno, Dr. Dipl.-Landw.**
**Ruedigerstrasse 13**
**D-6701 Otterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 121 129 B1

Jouve, 18, rue St-Denis, 75001 Paris, France

**Beschreibung**

Die Erfindung betrifft 2,5-Dihydro-5-aryliminopyrrolderivate, Verfahren zu ihrer Herstellung, sowie Herbizide, die diese Verbindungen als Wirkstoffe enthalten.

Es wurde gefunden, daß 2,5-Dihydro-5-arylimino-pyrrolderivate der Formel I

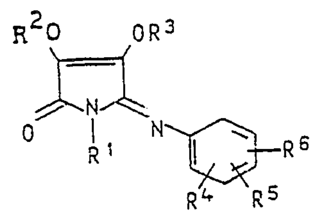

(I)

in der

$R^1$ Wasserstoff oder $C_1$-$C_4$-Alkyl,

$R^2$ $C_1$-$C_4$-Alkyl,

$R^3$ $C_1$-$C_4$-Alkyl und

$R^4$, $R^5$, $R^6$ unabhängig voneinander Wasserstoff, Cyano, Halogen, $C_1$-$C_4$-Alkyl, $C_1$-$C_4$-Alkoxy, $C_1$-$C_4$-Halogenalkyl und $C_1$-$C_4$-Halogenalkoxy bedeuten, herbizid wirksam sind.

Die Reste $R^1$, $R^2$ und $R^3$ in der allgemeinen Formel I stehen unabhängig voneinander für unverzweigte oder verzweigte $C_1$-$C_4$-Alkylreste, beispielsweise für Methyl, Ethyl, i-Propyl, n-Butyl, s-Butyl, i-Butyl, vorzugsweise für Methyl, Ethyl oder i-Propyl.

Diese Reste $R^4$, $R^5$ und $R^6$ können Wasserstoff oder bis zu drei gleiche oder verschiedene Substituenten, ausgewählt aus der Gruppe bestehend aus Cyano, Halogen, $C_1$-$C_4$-Alkyl, $C_1$-$C_4$-Alkoxy, $C_1$-$C_4$-Halogenalkyl und $C_1$-$C_4$-Halogenalkoxy sein. Der 5-Aryliminorest kann z. B. als Arylteil einen Phenyl-, Fluorphenyl-, Difluorphenyl-, Chlorphenyl-, Dichlorphenyl-, Bromphenyl-, Dibromphenyl-, Trichlorphenyl-, Trifluormethylphenyl-, Difluormethylphenyl-, Methoxyphenyl-, Ethoxyphenyl-, i-Propoxyphenyl-, Tolyl-, Difluormethoxyphenyl-, Trifluormethoxyphenyl-, Tetrafluorethoxyphenyl-, Cyanophenylrest enthalten. Die Phenylreste tragen ggf. die Substituenten in 2-, 3-, 4-, 2,4-, 3,4-, 3,5- oder 2,4,6-Stellung.

Bevorzugte 2,5-Dihydro-5-arylimino-pyrrolderivate sind Verbindungen der Formel I, wobei $R^1$ Wasserstoff oder Methyl, $R^2$ und $R^3$ Methyl und Ar einen gegebenenfalls durch Halogen, $C_1$-$C_4$-Alkyl, $C_1$-$C_4$-Halogenalkyl oder $C_1$-$C_4$-Halogenalkoxy einfach oder zweifach substituierten Phenylrest bedeutet. Bevorzugte Halogensubstituenten für die Phenylreste sind Fluor und Chlor.

Es wurde außerdem gefunden, daß man 2,5-Dihydro-5-arylimino-pyrrolderivate der Formel

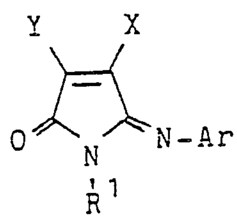

(Ia)

in der

$R^1$ Wasserstoff,

X Alkyl, Alkoxy, Dialkylamino, Phenyl oder substituiertes Phenyl,

Y Wasserstoff, Halogen oder Alkoxy und

Ar einen gegebenenfalls substituierten Arylrest bedeuten,

dadurch erhält, daß man Pyridazinone der Formel

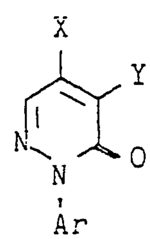

(IIa)

in der X, Y und Ar die obengenannten Bedeutungen haben, in Gegenwart eines inerten Lösungsmittels mit einer Base bei einer Temperatur zwischen 0 und 100 °C in ein 2,5-Dihydro-5-arylimino-pyrrolderivat

der Formel Ia überführt.

Eine vergleichbare Reaktion, welche eine Pyrazinonstruktur unter Spaltung der N-N-Bindung in ein Iminopyrrol überführt, ist in der Literatur bislang nicht beschrieben. Alle bisher am Pyridazinon beobachteten Ringverengungsreaktionen verlaufen unter Erhalt der N-N-Bindung und ergeben Pyrazolderivate (DE-AS 12 29 095 ; DE-AS 11 33 383 ; Chem. Pharm. Bull. 19, 1635 (1971) ; Chem. Pharm. Bull. 20, 747 (1972) ; Tetrahedron Letters 19, 1507 (1971) ; Chem. Pharm. Bull. 22, 229 (1974)). Die neue, unter milden Bedingungen mit sehr guten Ausbeuten verlaufende Reaktion zur Herstellung der 2,5-Dihydro-5-arylimino-pyrrolderivate der Formel Ia muß somit als sehr überraschend angesehen werden.

Die Ringverengung des Pyridazinons der Formel IIa zum 2,5-Dihydro-pyrrol-2-on verläuft in Gegenwart einer Base. Geeignete Basen sind beispielsweise Alkalimetallhydroxide, Alkalimetallalkoholate oder Natriumhydrid, wie Natriumhydroxid, Kaliumhydroxid, Natriummethylat, Kaliummethylat, Natriumethylat.

Die Menge an Base, bezogen auf 1 Mol Pyridazinon der Formel II, beträgt dabei 0,5 bis 1,5, vorzugsweise 1,0 bis 1,1 Mol.

Als inerte Lösungsmittel kommen vor allem dipolare, aprotische Lösungsmittel, wie Dimethylsulfoxid, Dimethylformamid, Dimethylacetamid oder Sulfolan, in Betracht.

Zur Alkylierung von in 1-Stellung unsubstituierten 2,5-Dihydro-5-arylimino-3,4-dialkoxypyrrol-2-onen können die üblichen Alkylierungsmittel, wie Alkylhalogenide, Alkylsulfate oder Alkyltosylate, verwendet werden. Die Alkylierung wird in an sich üblicher Weise durchgeführt.

Die Umsetzung wird zweckmäßigerweise so durchgeführt, daß das Pyridazinon der Formel IIa in einem inerten Lösungsmittel vorgelegt und unter Rühren mit der Base versetzt wird. Der Temperaturbereich der Reaktion liegt zwischen 0 und 100 °C, vorzugsweise zwischen 20 und 50 °C. Die erforderliche Zeit richtet sich vor allem nach dem Lösungsmittel und der Base und beträgt etwa 1 bis 50 Stunden.

Zur Aufarbeitung wird das Reaktionsgemisch in Wasser eingerührt und neutral gestellt. Die Endprodukte der Formel Ia mit $R^1$ = Wasserstoff können dann abgesaugt oder extrahiert werden. Zur Herstellung der alkylierten Produkte der Formel Ia mit $R^1$ = $C_1$-$C_4$-Alkyl wird dem Reaktionsgemisch nach beendeter Umlagerung (vor dem Einrühren in Wasser) ein Alkylierungsmittel zugesetzt. Nach wiederum 1 bis 20 Stunden Reaktionszeit bei einer Temperatur zwischen 0 und 100 °C kann wie oben beschrieben aufgearbeitet werden.

Die Substituenten in Formel Ia können folgende Bedeutung haben : X steht für $C_1$-$C_4$-Alkyl, $C_1$-$C_{10}$-Alkoxy, vorzugsweise $C_1$-$C_4$-Alkoxy, Dialkylamino mit $C_1$-$C_{10}$-Alkylresten, vorzugsweise mit $C_1$-$C_4$-Alkylresten, Phenyl oder durch Halogen, $C_1$-$C_4$-Alkyl, $C_1$-$C_4$-Alkoxy oder $C_1$-$C_4$-Halogenalkyl substituiertes Phenyl, Y steht für Wasserstoff, Halogen, vorzugsweise Fluor, Chlor, Brom, oder $C_1$-$C_{10}$-Alkoxy, vorzugsweise $C_1$-$C_4$-Alkoxy, und Ar steht für einen Phenyl- oder Naphthylrest, der durch Halogen, $C_1$-$C_4$-Alkyl, $C_1$-$C_4$-Alkoxy oder $C_1$-$C_4$-Halogenalkyl substituiert sein kann.

Beispiele für die Substituenten $R^1$ und Ar sind die für die entsprechenden Substituenten in Formel I genannten Reste. Beispiele für X sind Methyl, Ethyl, i-Propyl, n-Butyl, s-Butyl, i-Butyl, Methoxy, Ethoxy, n-Propoxy, i-Butoxy, s-Butoxy, Dimethylamino, Diethylamino, Di-n-butyl-amino, Phenyl, Fluorphenyl-, Difluorphenyl-, Chlorphenyl-, Dichlorphenyl-, Bromphenyl-, Dibromphenyl-, Trichlorphenyl-, Trifluormethylphenyl-, Difluormethyl-phenyl-, Methoxyphenyl-, Ethoxyphenyl-, i-Propoxyphenyl- oder Tolylreste. Biespiele für Y sind Wasserstoff, Fluor, Chlor, Brom, Methoxy, Ethoxy, i-Propoxy oder n-Butoxy.

Die folgenden Beispiele erläutern die Herstellung der 2,5-Dihydro-5-arylimino-pyrrolderivate der Formel I bzw. Ia. Dabei verhalten sich Gewichtsteile zu Volumenteilen wie Kilogramm zu Liter.

## Beispiel 1

12 Gewichtsteile 2-Phenyl-4,5-dimethoxy-pyridazin-3-on werden in 150 Volumenteilen Dimethylsulfoxid suspendiert und tropfenweise mit 9 Gewichtsteilen 30 %iger Natriummethylatlösung im Methanol versetzt. Nach 20 stündigem Rühren bei Raumtemperatur wird in 1 l Eiswasser eingerührt, durch Zugabe von konz. Salzsäure neutralgestellt und abgesaugt. Man erhält 11,4 Gewichtsteile 2,5-Dihydro-3,4-dimethoxy-5-phenyliminopyrrol-2-on vom Schmelzpunkt 141-143 °C (Verbindung Nr. 1).

## Beispiel 2

30,1 Gewichtsteile 2-(3,4-Dichlorphenyl)-4,5-dimethoxy-pyridazin-3-on werden in 200 Volumenteilen Dimethylsulfoxid suspendiert und bei Raumtemperatur tropfenweise mit 18 Gewichtsteilen 30 %iger Natriummethylatlösung in Methanol versetzt. Nach 20 stündigem Rühren werden 14,2 Gewichtsteile Methyliodid zugetropft. Nach wiederum mehrstündigem Rühren wird in 1 l Eiswasser gegeben, und der anfallende Niederschlag wird abgesaugt. Man erhält 29,4 Gewichtsteile 1-Methyl-2,5-dihydro-3,4-dimethoxy-5-(3,4-dichlorphenylimino)-pyrrol-2-on vom Schmelzpunkt 111-113 °C (Verbindung Nr. 53).

## Beispiel 3

12,4 Gewichtsteile 2,5-Diphenyl-pyridazin-3-on werden in 100 Volumenteilen Dimethylsulfoxid suspendiert und tropfenweise mit 9 Gewichtsteilen einer 30 %igen Natriummethylatlösung in Methanol

versetzt. Nach 20 stündigem Rühren wird in 500 Gewichtsteile Eiswasser gegeben und mit Salzsäure neutralgestellt. Das ausfallende Produkt wird abgesaugt und getrocknet, anschließend zur Reinigung über eine Kieselgelsäule (Laufmittel : Methylenchlorid) gegeben. Man erhält 8,7 Gewichtsteile 2,5-Dihydro-4-phenyl-5-phenylimino-pyrrol-2-on vom Schmelzpunkt 180-183 °C (Verbindung Nr. 62).

Beispiel 4

26 Gewichtsprozent 2-Phenyl-4-chlor-5-dimethylamino-pyridazin-3-on werden in 200 ml Dimethylsulfoxid vorgelegt und tropfenweise mit 18 Gewichtsteilen einer 30 %igen Lösung von Natriummethylat in Methanol versetzt. Nach 20 stündigem Rühren bei Raumtemperatur wird in 500 ml Eiswasser eingerührt und mit Salzsäure neutralgestellt. Das erhaltene Produkt wird abgesaugt. Ausbeute : 11,4 Gewichtsteile 2,5-Dihydro-3-chlor-4-dimethylamino-5-phenylimino-pyrrol-2-on mit dem Schmelzpunkt 135-138 °C (Verbindung Nr. 68).

Beispiel 5

18 g einer 30 %igen Lösung von Natriummethylat in Methanol werden in 150 ml Dimethylsulfoxid bei 70 °C am Rotationsverdampfer vom Methanol befreit. Die erhaltene Suspension wird portionsweise zu einer Suspension von 23,7 Gewichtsteilen 2-Phenyl-4-chlor-5-methoxy-pyridazin-3-on in 150 Volumenteilen Dimethylsulfoxid zugefügt. Nach mehrstündigem Rühren bei Raumtemperatur wird in 500 Gewichtsteile Eiswasser eingerührt und mit Salzsäure neutralgestellt. Man erhält 19,2 Gewichtsteile 2,5-Dihydro-3-chlor-4-methoxy-5-phenylimino-pyrrol-2-on vom Schmelzpunkt 179-180 °C (aus Toluol ; Verbindung Nr. 70).

Die folgenden Verbindungen der Formel Ia bzw. I lassen sich auf analogem Wege herstellen.

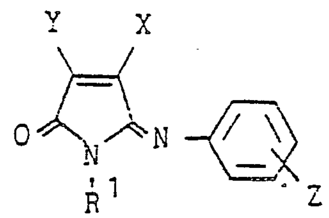

| Verbindung Nr. | $R^1$ | Y | X | Z | Fp [°C] |
|---|---|---|---|---|---|
| 1 | H | $OCH_3$ | $OCH_3$ | H | 141 - 143 |
| 2 | H | $OCH_3$ | $OCH_3$ | 3-$CH_3$ | 109 - 110 |
| 3 | H | $OCH_3$ | $OCH_3$ | 3-$OCF_2CHF_2$ | 110 - 111 |
| 4 | H | $OCH_3$ | $OCH_3$ | 3-$CF_3$ | 107 - 110 |
| 5 | H | $OCH_3$ | $OCH_3$ | 3-Br | 108 - 110 |
| 6 | H | $OCH_3$ | $OCH_3$ | 3-CN | 140 - 143 |
| 7 | H | $OCH_3$ | $OCH_3$ | 3-$CHF_2$ | 100 - 103 |
| 8 | H | $OCH_3$ | $OCH_3$ | 3-$OCHF_2$ | |
| 9 | H | $OCH_3$ | $OCH_3$ | 3-$OCF_2CHFCl$ | |
| 10 | H | $OCH_3$ | $OCH_3$ | 3-Cl | |
| 11 | H | $OCH_3$ | $OCH_3$ | 3-F | |
| 12 | H | $OCH_3$ | $OCH_3$ | 3-$OCH_3$ | 103 - 105 |
| 13 | H | $OCH_3$ | $OCH_3$ | 2-F | 146 - 148 |
| 14 | H | $OCH_3$ | $OCH_3$ | 2-$OCHF_2$ | 114 - 116 |
| 15 | H | $OCH_3$ | $OCH_3$ | 2-$CH_3$ | |
| 16 | H | $OCH_3$ | $OCH_3$ | 2-$OCH_3$ | |
| 17 | H | $OCH_3$ | $OCH_3$ | 4-F | |
| 18 | H | $OCH_3$ | $OCH_3$ | 4-Cl | |

(Fortsetzung)

| Verbindung Nr. | $R^1$ | Y | X | Z | Fp [°C] |
|---|---|---|---|---|---|
| 19 | H | $OCH_3$ | $OCH_3$ | 4-$CH_3$ | |
| 20 | H | $OCH_3$ | $OCH_3$ | 4-$OCH_3$ | |
| 21 | H | $OCH_3$ | $OCH_3$ | 3,4-$Cl_2$ | 133 - 135 |
| 22 | H | $OCH_3$ | $OCH_3$ | 3,5-$Cl_2$ | 156 - 158 |
| 23 | H | $OCH_3$ | $OCH_3$ | 2,4-$Cl_2$ | |
| 24 | H | $OCH_3$ | $OCH_3$ | 2,6-$Cl_2$ | |
| 25 | H | $OCH_3$ | $OCH_3$ | 2,4,6-$Cl_3$ | |
| 26 | H | $OCH_3$ | $OCH_3$ | 2,4-$F_2$ | 128 - 129 |
| 27 | H | $OCH_3$ | $OCH_3$ | 3,4-$F_2$ | 141 - 143 |
| 28 | H | $OCH_3$ | $OCH_3$ | 3-Cl, 4-F | 151 - 153 |
| 29 | H | $OCH_3$ | $OCH_3$ | 3-$CF_3$, 4-F | 150 - 153 |
| 30 | H | $OCH_3$ | $OCH_3$ | 3-Cl, 4-$OCHF_2$ | 118 - 120 |
| 31 | H | $OCH_3$ | $OCH_3$ | 3,5-$(CH_3)_2$ | |
| 32 | H | $OCH_3$ | $OCH_3$ | 3-$C_2H_5$ | |
| 33 | H | $OCH_3$ | $OCH_3$ | 3-$CH_3$, 4-F | 133 - 136 |
| 34 | H | $OCH_3$ | $OCH_3$ | 4-$CH(CH_3)_2$ | |
| 35 | H | $OC_2H_5$ | $OC_2H_5$ | H | |
| 36 | $CH_3$ | $OCH_3$ | $OCH_3$ | H | 57 - 58 |
| 37 | $CH_3$ | $OCH_3$ | $OCH_3$ | 3-$CF_3$ | |
| 38 | $CH_3$ | $OCH_3$ | $OCH_3$ | 3-Cl | |
| 39 | $CH_3$ | $OCH_3$ | $OCH_3$ | 3-F | |
| 40 | $CH_3$ | $OCH_3$ | $OCH_3$ | 3-Br | 99 - 101 |
| 41 | $CH_3$ | $OCH_3$ | $OCH_3$ | 3-CN | |
| 42 | $CH_3$ | $OCH_3$ | $OCH_3$ | 3-$CHF_2$ | 77 - 79 |
| 43 | $CH_3$ | $OCH_3$ | $OCH_3$ | 3-$OCH_3$ | |
| 44 | $CH_3$ | $OCH_3$ | $OCH_3$ | 3-$OCHF_2$ | |
| 45 | $CH_3$ | $OCH_3$ | $OCH_3$ | 3-$OCF_2CHF_2$ | |
| 46 | $CH_3$ | $OCH_3$ | $OCH_3$ | 2-F | |
| 47 | $CH_3$ | $OCH_3$ | $OCH_3$ | 2-$OCHF_2$ | |
| 48 | $CH_3$ | $OCH_3$ | $OCH_3$ | 2-$CH_3$ | |
| 49 | $CH_3$ | $OCH_3$ | $OCH_3$ | 4-F | |
| 50 | $CH_3$ | $OCH_3$ | $OCH_3$ | 4-Cl | |
| 51 | $CH_3$ | $OCH_3$ | $OCH_3$ | 4-$OCH_3$ | |
| 52 | $CH_3$ | $OCH_3$ | $OCH_3$ | 4-$CH_3$ | |
| 53 | $CH_3$ | $OCH_3$ | $OCH_3$ | 3,4-Cl | 111 - 113 |
| 54 | $CH_3$ | $OCH_3$ | $OCH_3$ | 3,5-$Cl_2$ | 100 - 102 |
| 55 | $CH_3$ | $OCH_3$ | $OCH_3$ | 2,4-$Cl_2$ | |
| 56 | $CJ_3$ | $OCH_3$ | $OCH_3$ | 2,4-$F_2$ | |
| 57 | $CH_3$ | $OCH_3$ | $OCH_3$ | 3,4-$F_2$ | 101 - 103 |
| 58 | $CH_3$ | $OCH_3$ | $OCH_3$ | 3,5-$(CH_3)_2$ | |
| 59 | i-$C_3H_7$ | $OCH_3$ | $OCH_3$ | H | |

(Fortsetzung)

| Verbindung Nr. | $R^1$ | Y | X | Z | Fp [°C] |
|---|---|---|---|---|---|
| 60 | $CH_3$ | $OC_2H_5$ | $OC_2H_5$ | H | |
| 61 | $C_2H_5$ | $OCH_3$ | $OCH_3$ | H | |
| 62 | H | H | $C_6H_5$ | H | 180 - 183 |
| 63 | H | Cl | $C_6H_5$ | H | |
| 64 | H | H | $CH_3$ | H | |
| 65 | H | Cl | $CH_3$ | H | |
| 66 | H | Br | $CH_3$ | H | |
| 67 | H | H | $OCH_3$ | H | 156 - 159 |
| 68 | H | Cl | $N(CH_3)_2$ | H | 135 - 138 |
| 69 | H | Cl | $N(C_2H_5)_2$ | H | |
| 70 | H | Cl | $OCH_3$ | H | 179 - 180 |
| 71 | H | F | $OCH_3$ | H | |
| 72 | H | Br | $OCH_3$ | H | |

Die 2,5-Dihydro-5-arylimino-pyrrolderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 46 mit 10 Gewichtsteilen N-Methyl-α-pyrrolidon und erhält eine Lösung, die zur Anwendung in Form kleinster Tropfen geeignet ist.

II. 10 Gewichtsteile der Verbindung Nr. 3 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 1 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 36 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile der Verbindung Nr. 22 werden mit 3 Gewichtsteilen des Natriumsalzes der Diisobutyl-naphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung Nr. 33 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 27 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 57 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe bei Nachauflaufanwendung für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium, 0,05 bis 5 kg/ha, vorzugsweise 0,25 bis 3 kg/ha.

Die herbizide Wirkung der 2,5-Dihydro-5-arylimino-pyrrolderivate der Formel I auf Pflanzen aus der Familie der Gräser (Gramineen) läßt sich durch Gewächshausversuche zeigen :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dieser Applikationsmethode beträgt die Anfwandmenge 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden entweder direkt gesäte und in den gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,5 bzw. 3,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Amaranthus spp. (Fuchsschwanz), Avena sativa (Hafer), Cassia tora, Centaurea cyanus (Kornblume), Chenopodium album (Weißer Gänsefuß), Galium aparine (Klettenlabkraut), Gossypium hirsutum (Baumwolle), Ipomoea spp. (Prunkwindearten), Mercurialis annua (Einjähriges Bingelkraut), Sinapis alba (Weißer Senf), Zea mays (Mais).

Bei Vorauflaufanwendung zeigen beispielsweise die Verbindungen Nr. 1 und 36 sowie die Verbindung Nr. 3 eine beachtliche herbizide Wirkung, wobei sich bei letzterer eine Selektivität für Hafer zeigt.

Im Nachauflaufverfahren zeigen beispielsweise die Verbindungen Nr. 3, 7, 22, 27, 33, 36, 46 und 57 bei einer Aufwandmenge von 3,0 kg/ha gegenüber breitblättrigen unerwünschten Pflanzen eine herbizide Wirkung. Die Verbindung Nr. 1 bekämpft Unkräuter mit beispielsweise 0,5 kg Wirkstoff/ha selektiv bzw. mit nur geringer Schädigung an Baumwolle und Mais.

In Anbetracht der Verträglichkeit und der Vielseitigkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum | |
| (Gossypium arboreum) | |
| Gossypium herbaceium | Baumwolle |
| (Gossypium vitifolium) | |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum | Tabak |
| (N. rustica) | |

| Botanischer Name | Deutscher Name |
|---|---|
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum | Wurzelpetersilie |
| spp. tuberosum | |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Tibes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 2,5-Dihydro-5-arylimino-pyrrolderivate der Formel I bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, Cyclohexan-1,3-dion-derivate und andere in Betracht, beispielsweise

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-(pyridino-[3,2-e]2,1,3-thiadiazin-(4)-on-2,2-dioxid
1-Cyan-3-(1-methylethyl)-2,1,3-benzothiadiazin-4(3H)-on-2,2-dioxid
N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-β-chlorethyl-2,6-dinitro-4-trifluormethyl-anilin
N-n-Propyl-N-cyclopropylmethyl-2,6-dinitro-4-trifluor-methyl-anilin

N,N-Di-n-propyl-2,6-dinitro-3-amino-4-trifluormethylanilin
N,N-Di-n-propyl-2,6-dinitro-4-methyl-anilin N,N-Di-n-propyl-2,6-dinitro-4-methylsulfonyl-anilin
N,N-Di-n-propyl-2,6-dinitro-4-aminosulfonyl-anilin
N,N-Di-beta-chlorethyl-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert.butyl-4-methylphenyl-ester
N-Phenylcarbaminsäure-isopropylester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
$3^{\alpha}$-N-isopropyl-carbamoyloxy-propionanilid

Ethyl-N-[3-($N^{\alpha}$-phenylcarbamoyloxy)-phenyl]-carbamat
Methyl-N-[3-($N^{\alpha}$-methyl-$N^{\alpha}$-phenylcarbamoyloxy)-phenyl]-carbamat
Isopropyl-N-[3-($N^{\alpha}$-ethyl-$N^{\alpha}$-phenylcarbamoyloxy)-phenyl]-carbamat
Methyl-N-[3-($N^{\alpha}$-3-methylphenylcarbamoyloxy)-phenyl]-carbamat
Ethyl-N-[3-($N^{\alpha}$-3-chlor-4-fluorphenylcarbamoyloxy)-phenyl]-carbamat
Ethyl-N-[3-($N^{\alpha}$-3,4-difluorphenylcarbamoyloxy)-phenyl]-carbamat

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiocarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec. butyl-thiolcarbaminsäure-ethylester
N,N-Di-sec. butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester
S-Ethyl-hexahydro-1H-azepin-1-carbothiolat
S-Ethyl-3-methylhexahydro-1-H-azepin-1-carbothiolat

N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz
Alpha,alpha,beta,beta-Tetrafluorpropionsäure-Natriumsalz
Alpha-Methyl-alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-($4^{\alpha}$-Chlorphenoxy)-phenoxy]-propionsäureisobutylester
2-[4-($2^{\alpha}$,$4^{\alpha}$-Dichlorphenoxy)-phenoxy]-propionsäuremethylester
2-[4-($4^{\alpha}$-Trifluormethylphenoxy)-phenoxy]-propionsäure-methylester

2-[4-(2$^{\alpha}$-Chlor-4$^{\alpha}$-trifluorphenoxy)-phenoxy]-propionsäure-Natriumsalz
2-[4-(3$^{\alpha}$,5$^{\alpha}$-Dichlorpyridyl-2-oxy)-phenoxy]-propionsäure-Natriumsalz

2-(N-Benzoyl-N-(3,4-dichlorphenyl)-amino)-propionsäureethylester
2-(N-Benzoyl-3-chlor-4-fluorphenylamino)-propionsäure-methylester
2-(N-Benzoyl-3-chlor-4-fluorphenylamino)-propionsäure-isopropylester
2-Chlor-4-ethylamino-6-isopropylamino-1,3,5-triazin
2-Chlor-4-ethylamino-6-(amino-2$^{\alpha}$-propionitril)-1,3,5-triazin
2-Chlor-4,6-bisethylamino-1,3,5-triazin
2-Chlor-4,6-bisisopropylamino-1,3,5-triazin
3-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
3-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin

2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-Isopropyl-5-brom-6-methyluracil
3-sec.Butyl-5-brom-6-methyluracil
3-Cyclohexyl-5,6-trimethylenuracil

2-Methyl-4-(3$^{\alpha}$-trifluormethylphenyl)-tetrahydro-1,2,4-oxadiazin-3,5-dion
2-Methyl-4-(4$^{\alpha}$-fluorphenyl)-tetrahydro-1,2,4-oxadiazin-3,5-dion
3-Amino-1,2,4-triazol

1-(4-Chlorphenoxy-3,3-dimethyl-1-(H-1,2,4-triazolyl)-2-butanon
N,N-Diallylchloracetamid
N-Isopropyl-2-chloracetanilid
N-(1-Methyl-propin-2-yl)-2-chloracetanilid
2-Methyl-6-ethyl-N-ethoxymethyl-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracet-anilid
2-Methyl-6-ethyl-N-(pyrazolyl-methyl)-2-chloracetanilid
2,6-Dimethyl-N-(pyrazolyl-methyl)-2-chloracetanilid
2,6-Dimethyl-N-(4-methylpyrazolyl-methyl)-2-chloracetanilid
2,6-Dimethyl-N-(1,2,4-triazolyl-methyl)-2-chloracetanilid
2,6-Dimethyl-N-(3,5-dimethylpyrazolyl-methyl)-2-chloracetanilid
2,6-Dimethyl-N-(1,3-dioxalan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3-Dimethyl-N-isopropyl-2-chloracetanilid
2,6-Diethyl-N-(2-n-propoxy-ethyl)-2-chloracetanilid

alpha-(2-Methyl-4-chlorphenoxy)-N-methoxy-acetamid
2-(alpha-Naphthoxy)-N,N-diethylpropionamid
2,2-Diphenyl-N,N-dimethylacetamid
alpha-(3,4,5-Tribrompyrazolyl)-N,N-dimethylpropionamid
N-(1,1-Dimethylprop-2-inyl)-3,5-dichlorbenzamid
N-Naphth-1-yl-phthalamidsäure

Propionsäure-3,4-dichloranilid
Cyclopropancarbonsäure-3,4-dichloranilid

Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
0-(Methylaminosulfonyl)-glykolsäure-hexamethylenimid
2,6-Dichlor-thiobenzamid
2,6-Dichlorbenzonitril

3,5-Dibrom-4-hydroxy-benzonitril (Salze)
3,5-Diiod-4-hydroxy-benzonitril (Salze)
3,5-Dibrom-4-hydroxy-0-2,4-dinitrophenylbenzaldoxim (Salze)
Pentachlorphenyl-Natriumsalz
2,4-Dichlorphenyl-4$^{\alpha}$-nitrophenylether
2,4,6-Trichlorphenyl-4$^{\alpha}$-nitrophenylether
2-Fluor-4,6-dichlorphenyl-4$^{\alpha}$-nitrophenylether
2-Chlor-4-trifluormethylphenyl-4$^{\alpha}$-nitrophenylether
2,4$^{\alpha}$-Dinitro-4-trifluormethyl-diphenylether
2,4-Dichlorphenyl-3$^{\alpha}$-methoxy-4$^{\alpha}$-nitro-phenylether
2-Chlor-4-trifluormethylphenyl-3$^{\alpha}$-ethoxy-4$^{\alpha}$-nitro-phenylether
2-Chlor-4-trifluormethylphenyl-3$^{\alpha}$-carboxy-4$^{\alpha}$-nitro-phenylether (Salze)
2,4-Dichlorphenyl-3$^{\alpha}$-methoxycarbonyl-4$^{\alpha}$-nitro-phenylether
2-(3,4-Dichlorphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
3-(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Methyl-4,6-dinitrophenol (Salze, Ester)
3-(4-Chlorphenyl)-3,4,5,9,10-pentaazatetracyclo-[5,4,1,$0^{2,6}$,$0^{8,1}$]-dodeca-3,9-dien
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(alpha,alpha-Dimethylbenzyl)-3-(4-methylphenyl)-harnstoff
1-Phenyl-3-(2-methylcyclohexyl)-harnstoff
1-(4-Chlorphenyl)-3,3-dimethyl-harnstoff
1-(4-Chlorphenyl)-3-methyl-3-(butin-1-yl-3)-harnstoff
1-(3,4-Dichlorphenyl)-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(alpha,alpha,beta,beta-Tetrafluorethoxyphenyl)-3,3-dimethyl-harnstoff

1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-[4(4$^{\alpha}$-Chlorphenoxy)-phenyl]-3,3-dimethyl-harnstoff
1-[4(4$^{\alpha}$-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff Imidazolidin-2-on-1-carbonsäure-isobutylamid
1,2-Dimethyl-3,5-diphenylpyrazolium-methylsulfat

1,3-Dimethyl-4-(3,4-dichlorbenzoyl)-5-(4-methylphenylsulfonyloxy)-pyrazol 2,3,5-Trichlor-pyridinol-(4)
1-Methyl-3-phenyl-5-(3$^{\alpha}$-trifluormethylphenyl)-pyridon-(4)
1-Methyl-4-phenyl-pyridiniumchlorid
1,1-Dimethylpyridiniumchlorid
1,1$^{\alpha}$-Dimethyl-4,4$^{\alpha}$-dipyridylium-di(methylsulfat)
1,1$^{\alpha}$-Di(3,5-dimethylmorpholin-carbonylmethyl)-4,4$^{\alpha}$-dipyridylium-dichlorid 1,1$^{\alpha}$-Ethylen-2,2$^{\alpha}$-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2,4-Dichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

Alpha-Naphthoxyessigsäuremethylester
2-(2,4-Dichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester) Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
0,0-Di-n-butyl-(1-n-butylamino-cyclohexyl)-phosphonat
Trithiobutylphosphit
0,0-Diisopropyl-5-(2-benzolsulfonylamino-ethyl)-phosphordithioat
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N,N-dimethylhydrazid (Salze)
(2-Chlorethyl)-trimethyl-ammoniumchlorid
(2-Methyl-4-phenylsulfonyl)-trifluormethansulfonanilid

Ammoniumrhodanid
Calciumcyanamid

2-Chlor-4-trifluormethylphenyl-3$^{\alpha}$-ethoxycarbonyl-4$^{\alpha}$-nitrophenylether
1-(4-Benzyloxyphenyl)-3-methyl-3-methoxyharnstoff
2-[1-(2,5-Dimethylphenyl)-ethylsulfonyl]-pyridin-N-oxid
3-tert.-Butylamino-4-methoxycarbonyl-5-methylpyrazol
N-Benzyl-N-isopropyl-trimethylacetamid
2-[4-(4$^{\alpha}$-Chlorphenoxymethyl)-phenoxy]-propionsäuremethylester
2-[4-(5$^{\alpha}$-Brompyridyl-2-oxy)-phenoxy]-propionsäureethylester
2-[4-(5$^{\alpha}$-Iodpyridyl-2-oxy)-phenoxy]-propionsäure-n-butylester
2-Chlor-4-trifluormethylphenyl-3$^{\alpha}$-(2-fluorethoxy)-4$^{\alpha}$-nitro-phenylether
2-Chlor-4-trifluormethylphenyl-3$^{\alpha}$-ethoxycarbonylmethylthio-4$^{\alpha}$-nitrophenylether
2,4,6-Trichlorphenyl-3$^{\alpha}$-ethoxycarbonyl)methylthio-4$^{\alpha}$-nitrophenylether
2-[1-(N-ethoxyamino)-butyliden]-5-(2-ethylthiopropyl)-3-hydroxy-cyclohexen-(2)-on-(1) (Salze)
2-[1-(N-ethoxyamino)-butyliden]-5-(2-phenylthiopropyl)-3-hydroxy-cyclohexen-(2)-on-(1) (Salze)

4-[4-(4$^{\alpha}$-Trifluormethyl)-phenoxy]-penten-2-carbonsäureethylester
2-Chlor-4-trifluormethyl-3$^{\alpha}$-methoxycarbonyl-4$^{\alpha}$-nitrophenylether
2,4-Dichlorphenyl-3$^{\alpha}$-carboxy-4$^{\alpha}$-nitrophenylether (Salze)
4,5-Dimethoxy-2-(3-alpha,alpha,beta-trifluor-beta-bromethoxyphenyl)-3-(2H)-pyridazinon
2,4-Dichlor-3$^{\alpha}$[2-(2-ethoxy-ethoxy)-ethoxy]-4$^{\alpha}$-nitro-diphenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzol-sulfonamid

1-(3-Chlor-4-ethoxyphenyl)-3,3-dimethylharnstoff
2-Methyl-4-chlorphenoxy-thioessigsäureethylester
2-Chlor-3,5-dijod-4-acetoxy-pyridin
1(-4-[2-(4-Methylphenyl)-ethoxy]-phenyl)-3-methyl-3-methoxyharnstoff
2,6-Dimethyl-N-(pyrazolyl-methylenoxymethyl)-2-chloracetanilid
2-Methyl-6-ethyl-N-(pyrazolyl-methylenoxymethyl)-2-chloracetanilid alpha-2,4-Dichlorphenoxy-propionsäure)-(3-methoxycarbonylamino)-anilid
1-(alpha-2-Brom-4-chlorphenoxypropionsäure)-3-(O-methylcarbamoyl)-anilid
2-Methyl-6-ethyl-N-(pyrazolyl-ethylenoxymethyl)-2-chloracetanilid

2-(3-Trifluormethylphenyl)-4H-3,1-benzoxazin-4-on
2-(3-Pentafluorethoxyphenyl)-4H-3,1-benzoxazin-4-on
2-(3-Trifluormethylthio-phenyl)-4H-3,1-benzoxazin-4-on
2-(3-Difluorchlormethoxyphenyl)-4H-3,1-benzoxazin-4-on

5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-alpha,alpha,beta,beta-tetrafluorethoxyphenyl)-4H-3,1-benzoxazin-4-on
5-Fluor-2-(3-alpha,alpha,beta,beta-tetrafluorethoxyphenyl)-4H-3,1-benzoxazin-4-on
5-Chlor-2-(4-difluorchlormethoxyphenyl)-4H-3,1-benzoxazin-4-on
5-Fluor-2-(4-difluorchlormethoxyphenyl)-4H-3,1-benzoxazin-4-on
5-Fluor-2-phenyl-4H-3,1-benzoxazin-4-on
5-Fluor-2-(3-difluormethoxyphenyl)-4H-3,1-benzoxazin-4-on
5-Chlor-2-phenyl-4H-3,1-benzoxazin-4-on

N-3-Chlor-4-isopropylphenyl-thiolcarbaminsäuremethylester
6-Methyl-3-methoxy-5,6-dihydro-1,2,4,6-thiatriazin-5-on-1,1-dioxid Natriumsalz
6-Methyl-3-ethoxy-5,6-dihydro-1,2,4,6-thiatriazin-5-on-1,1-dioxid
5-Amino-4-chlor-2-phenyl-3(2H)-pyridazinon
5-Amino-4-brom-2-phenyl-3(2H)-pyridazinon

5-Methylamino-4-chlor-2-(3-trifluormethylphenyl)-3(2H)-pyridazinon
5-Methylamino-4-chlor-2-(3-alpha,alpha,beta,beta-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
1-[3$^{\alpha}$-(2″-Chlor-4″-trifluormethylphenoxy)]-phenyl-4,5-dimethoxy-pyridazinon-6
1-[4$^{\alpha}$-3″-Trifluormethyl-phenoxy)]-phenyl-4,5-dimethoxy-pyridazinon-6
4,5-Dimethoxy-2-(3-alpha,alpha,beta-trifluor-beta-bromethoxyphenyl)-3-(2H)-pyridazinon
N-[4-(4$^{\alpha}$-Methoxy-phenoxy)-3-chlor-phenyl]-carbaminsäuremethylester
N-[4-(4$^{\alpha}$-Difluormethoxy-phenoxy)-3-chlor-phenyl]-thio-carbaminsäuremethylester
N-[4-(4$^{\alpha}$-Difluormethoxy-phenoxy)-phenyl]thio-carbaminsäuremethylester
1-[4-(4$^{\alpha}$-Methylphenylpropyl)-phenyl]-3-methyl-3-methoxyharnstoff
1-[3-(4$^{\alpha}$-Chlorphenyl-propyl)-phenyl]-3-methyl-3-methoxyharnstoff
1-[4-(3-Phenyl-2-methyl-propyl)-phenyl]-3-methyl-methoxyharnstoff
1-[4-(3-(4$^{\alpha}$-Chlorphenyl)-2-methyl-propyl)-phenyl]-3-methyl-3-methoxyharnstoff
1-[4-(3-(4$^{\alpha}$-Methylphenyl)-2-methylpropyl)-phenyl]-3-methyl-3-methoxyharnstoff
2-[1-(N-Ethyloxyamino)-butyliden]-5(4-ethylphenyl)-3-hydroxy-cyclohexen-(2)-on-(1) (Salze)
2-[1-(N-Ethyloxyamino)-butyliden]-5(4-fluorphenyl)-3-hydroxy-cyclohexen-(2)-on-(1) (Salze)
2-[1-(N-Ethyloxyamino)-butyliden]-5-(4-Chlorphenyl)-3-hydroxy-cyclohexen-(2)-on-(1) (Salze)
2$^{\alpha}$-(2,4,6-Trichlorphenyl)-hydrazino-2-cyanacrylsäuremethylester
2-[1-(N-Ethyloxamino)-butyliden]-5-(1,3,3-trimethyl-cyclohexen-1-yl-2)-3-hydroxy-cyclohexen-(2)-on-(1) (Salze)
2-[1-(N-Ethyloxamino)-butyliden]-5-(2,4,4-trimethyl-cyclohexen-1-yl-3)-3-hydroxy-cyclohexen-(2)-on-(1) (Salze)
2-[1-(N-3-Chlorallyl-oxamino-butyliden]-5-(1-methyl-cyclohex-1-en-4-yl)-3-hydroxy-cyclohexen-(2)-on-(1) (Salze)
3-Isobutoxy-5-methyl-4-methoxycarbonyl-pyrazol
5-Amino-1-(2,4,6-trichlorphenyl)-4-cyano-pyrazol
5-Amino-1-(2,4,6-tribromphenyl)-4-cyano-pyrazol
5-Amino-1-(2,4,6-trichlorphenyl)-4-methoxycarbonyl-pyrazol
5-Amino-(2,4-dichlor-6-bromphenyl)-4-methoxycarbonyl-pyrazol
5-Amino-(2,6-dichlor-4-bromphenyl)-4-methoxycarbonyl-pyrazol
5-Chlor-2-(3-trifluormethyl-phenyl)-4H-3,1-benzoxazin-4-on
5-Fluor-2-(3-trifluormethyl-phenyl)-4H-3,1-benzoxazin-4-on
2-(3-Tetrafluorethoxyphenyl)-4H-3,1-benzoxazin-4-on

5-Chlor-2-(4α-fluorphenyl)-4H-3,1-benzoxazin-4-on
5-Fluor-2-(4α-fluorphenyl)-4H-3,1-benzoxazin-4-on
5-Fluor-2-(3α-fluorphenyl)-4H-3,1-benzoxazin-4-on
5-Fluor-2-(3α-fluorphenyl)-4H-3,1-benzoxazin-4-on
5-Chlor-2-(3α-difluorchlormethylphenyl)-4H-3,1-benzoxazin-4-on
5-Fluor-2-(3α-difluorchlormethylphenyl)-4H-3,1-benzoxazin-4-on

6-Methyl-3-methoxy-5-(4α-nitrophenoxy)-6H-1,2,4,6-thiatriazin-1,1-dioxid
6-Methyl-3-methoxy-5-(propargyloxy-6H-1,2,4,6-thiatriazin-1,1-dioxid
6-Methyl-3-methoxy-5-(2,4-dichlorbenzoxy)-6H-1,2,4,6-thiatriazin-1,1-dioxid
2-(2α,4α-Dichlorphenoxy)-2-fluorpropionsäure (Salze, Ester)
2-[4-(5α-Trifluormethylpyridyl-2-oxy)-phenoxy]-propionsäurebutylester
2-[4-(3α-Chlor-5α-trifluormethylpyridyl-2-oxy)-phenoxy]-propionsäure (Salze, Ester)
2-[4-(6-Chlorchinoxalyl-2-oxy)-phenoxy]-propionsäure-pentylester
2-[4-(6-Chlor-chinoxalyl-2-oxy)-phenoxy]-propionsäuremethylester
2-[4-(6-Chlorbenzthiazolyl-2-oxy)-phenoxy]-propionsäure (Salze, Ester)
2-[4-(6-Chlorbenzoxazolyl-2-oxy)-phenoxy]-propionsäure (Salze, Ester)
1-[5-(3-Fluorbenzylthio)-thiadiazolyl-2]-1-methylharnstoff
2-Methoxycarbonyl-N-(3,5-dimethylpyrimidinyl-2-aminocarbonyl)-benzol-sulfonamid
alpha-(3,5,6-Trichlor-pyrid-2-yl-oxy)-essigsäure (Salze, Ester)
alpha-(4-Amino-3,5-dichlor-6-fluor-pyrid-2-yl-oxy)-essigsäure (Salze, Ester)
S-[N-(4-Chlorphenyl)-N-isopropyl-carbamoyl-methyl]-0,0-dimethyl-dithiophosphat
Ammonium-(3-amino-3-carboxy-propyl)-methylphosphinat(Hydroxy)-(methyl)-phosphinyl-L-alphaaminobutyryl-L-alanyl-Natriumsalz
4-Trifluormethyl-diphenylether
2-(3,5-Dichlorphenyl)-2-(2α2α2α-trichlorethyl)-oxiran
2,4-Diamino-5-methylthio-6-chlor-pyrimidin
N-(4-Ethylthio-2-trifluormethyl-phenyl)-methylsulfonamid
3-Methoxy-4-methyl-5(3-methyl-2-butenyloxy)-1,2-di(hydroxymethyl)-benzol
2-(3,5-Dimethylphenoxy)-2-(1,2,4-triazolyl-1)-essigsäure-N-tertiär-butylamid
2-(3,5-Dichlorphenoxy)-2-(1,2,4-triazolyl-1)-essigsäure-N-tertiär-butylamid
3,7-Dichlor-8-chinolincarbonsäure (Salze, Ester)
5-[2-Chlor-4-trifluormethyl-phenoxy-N-1-(methoxycarbonyl)-ethoxy]-2-nitrobenzamid
N-[3-(1-Ethyl-1-methylpropyl)-isoxazolyl-5]-2,6-dimethoxybenzamid
2α-Methoxyethyl-2-[5-(2-chlor-4-trifluormethyl-phenoxy)-2-nitrophenoxy]-propionat
Methyl-6-(4-isopropyl-4-methyl-5-oxo-2-imidazolin-2-yl)-3-methylbenzoat
Methyl-6-(4-isopropyl-4-methyl-5-oxo-2-imidozin-2-yl)-4-methylbenzoat Benzyltrimethylammoniumchlorid
1-[alpha-(Trifluormethyl-phenoxy)-phenoxy-propionsäure]-3-(0-methyl-carbamoyl)-anilid
1-Dodecyl-cycloheptan-2-on
N-[2-Chlor-4-(methylsulfonyl)-phenyl]-chlormethansulfonamid
N-[2-Brom-4-(ethylsulfonyl)-phenyl]-chlormethansulfonamid
N-[2,3-Dichlor-4-(ethylsulfonyl)-phenyl]-chlormethansulfonamid
2-[1-(N-Ethoxylamino)-pyropyliden-]-5-(pyrid-(3)yl)-3-hydroxy-cyclo-hexen-2-on (Salze)
2,4,5-Trichlorphenoxyessigsäure (Salze, Ester, Amide).

Außerdem kann es von Nutzen sein, die 2,5-Dihydro-5-arylimino-pyrrolderivate der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

## Patentansprüche

1. 2,5-Dihydro-5-arylimino-pyrrolderivate der Formel I

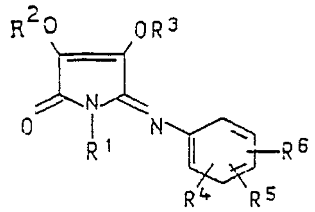

(I)

in der
$R^1$ Wasserstoff oder $C_1$-$C_4$-Alkyl,
$R^2$ $C_1$-$C_4$-Alkyl,
$R^3$ $C_1$-$C_4$-Alkyl und
$R^4$, $R^5$, $R^6$ unabhängig voneinander Wasserstoff, Cyano, Halogen, $C_1$-$C_4$-Alkyl, $C_1$-$C_4$-Alkoxy, $C_1$-$C_4$-Halogenalkyl und $C_1$-$C_4$-Halogenalkoxy bedeuten.

2. Verfahren zur Herstellung von 2,5-Dihydro-5-arylimino-pyrrolderivaten der Formel

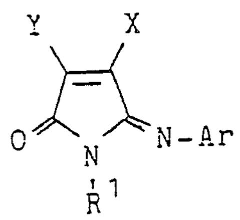

(Ia)

in der
$R^1$ Wasserstoff,
X Alkyl, Alkoxy, Dialkylamino, Phenyl oder substituiertes Phenyl,
Y Wasserstoff, Halogen oder Alkoxy und
Ar einen gegebenenfalls substituierten Arylrest bedeuten,
dadurch gekennzeichnet, daß man Pyridazinone der Formel

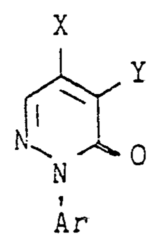

(IIa)

in der X, Y und Ar die obengenannten Bedeutungen haben, in Gegenwart eines inerten Lösungsmittels mit einer Base bei einer Temperatur zwischen 0 und 100 °C in ein 2,5-Dihydro-5-arylimino-pyrrolderivat der Formel Ia überführt.

3. Herbizid enthaltend ein 2,5-Dihydro-5-arylimino-pyrrolderivat der Formel I gemäß Anspruch 1.

4. Herbizid enthaltend inerte Zusatzstoffe und ein 2,5-Dihydro-5-arylimino-pyrrolderivat der Formel I gemäß Anspruch 1.

5. Verfahren zur Bekämpfung unerwünschten Pflanzenwuchses, dadurch gekennzeichnet, daß man die unerwünschten Pflanzen und/oder die von unerwünschtem Pflanzenwachstum freizuhaltenden Flächen mit einer herbizid wirksamen Menge eines Pyrrolderivates der Formel I gemäß Anspruch 1 behandelt.

## Claims

1. A 2,5-dihydro-5-aryliminopyrrole derivative of the formula I

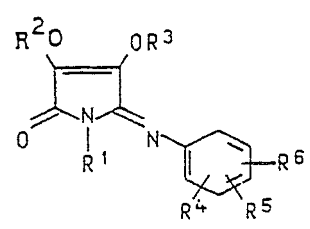

(I)

where
$R^1$ is hydrogen or $C_1$-$C_4$-alkyl,
$R^2$ is $C_1$-$C_4$-alkyl,
$R^3$ is $C_1$-$C_4$-alkyl and
$R^4$, $R^5$ and $R^6$ independently of one another are each hydrogen, cyano, halogen, $C_1$-$C_4$-alkyl, $C_1$-$C_4$-alkoxy, $C_1$-$C_4$-haloalkyl or $C_1$-$C_4$-haloalkoxy.

2. A process for the preparation of a 2,5-dihydro-5-aryliminopyrrole derivative of the formula

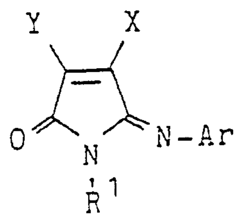

(Ia)

where $R^1$ is hydrogen,
X is alkyl, alkoxy, dialkylamino, phenyl or substituted phenyl,
Y is hydrogen, halogen or alkoxy and
Ar is unsubstituted or substituted aryl,
wherein a pyridazinone of the formula

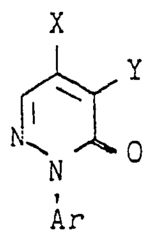

(IIa)

where X, Y and Ar have the abovementioned meanings, is converted with a base in the presence of an inert solvent at from 0 to 100 °C into a 2,5-dihydro-5-aryliminopyrrole derivative of the formula Ia.

3. A herbicide containing a 2,5-dihydro-5-aryliminopyrrole derivative of the formula I as claimed in claim 1.

4. A herbicide containing inert additives and a 2,5-dihydro-5-aryliminopyrrole derivative of the formula I as claimed in claim 1.

5. A method for controlling undesirable plant growth, wherein the undesirable plants and/or the areas to be kept free of undesirable plant growth are treated with a herbicidally effective amount of a pyrrole derivative of the formula I as claimed in claim 1.

**Revendications**

1. Dérivés de 2,5-dihydro-5-arylimino-pyrrole de formule I

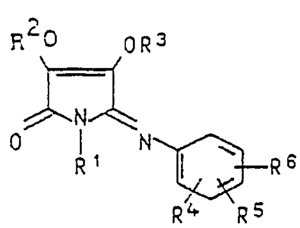

(I)

dans laquelle
$R^1$ représente hydrogène ou alkyle en $C_1$-$C_4$
$R^2$, alkyle en $C_1$-$C_4$
$R^3$, alkyle en $C_1$-$C_4$ et
$R^4$, $R^5$, $R^6$ représentent, indépendamment l'un de l'autre, hydrogène, cyano, halogène, alkyle en $C_1$-$C_4$, alcoxy en $C_1$-$C_4$, halogènalkyle en $C_1$-$C_4$ et halogénalcoxy en $C_1$-$C_4$.

2. Procédé de préparation de dérivés de 2,5-dihydro-5-aryl-iminopyrrole, de formule

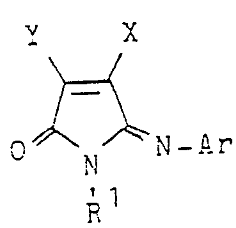

(Ia)

dans laquelle

$R^1$ représente hydrogène

X, alkyle, alcoxy, dialkylamino, phényle ou phényle substitué

Y, hydrogène, halogène ou alcoxy et

Ar un reste aryle éventuellement substitué

caractérisé par le fait que l'on transforme des pyridazinones de formule

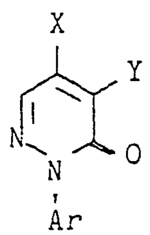

(IIa)

dans laquelle X, Y et Ar ont les significations susmentionnées en présence d'un solvant inerte, avec une base, à une température comprise entre 0 et 100 °C, en un dérivé de 2,5-dihydro-5-arylimino-pyrrole de formule Ia.

3. Herbicide, contenant un 2,5-dihydro-5-arylimino-pyrrole de formule I selon la revendication 1.

4. Herbicide contenant des additifs inertes et un dérivé de 2,5-dihydro-5-arylimino-pyrrole de formule I selon la revendication 1.

5. Procédé pour lutter contre la croissance des plantes indésirables, caractérisé par le fait qu'on traite les plantes indésirables et/ou les surfaces à protéger contre une croissance de plantes indésirables, avec une quantité efficace du point de vue herbicide d'un dérivé de pyrrole de formule I selon la revendication 1.